(19) Europäisches Patentamt
European Patent Office
Office européen des brevets

(11) EP 1 755 543 B1

(12) FASCICULE DE BREVET EUROPEEN

(45) Date de publication et mention de la délivrance du brevet:
**28.10.2015 Bulletin 2015/44**

(21) Numéro de dépôt: **05778721.0**

(22) Date de dépôt: **15.06.2005**

(51) Int Cl.:
***A61K 8/35*** (2006.01) ***A61K 8/37*** (2006.01)
***A61Q 17/04*** (2006.01)

(86) Numéro de dépôt international:
**PCT/FR2005/001503**

(87) Numéro de publication internationale:
**WO 2006/005846 (19.01.2006 Gazette 2006/03)**

# (54) COMPOSITION ANTI-DECOLORATION

ZUSAMMENSETZUNG ZUM SCHUTZ VOR FARBAUSBLEICHUNG

ANTI-COLOUR FADING COMPOSITION

(84) Etats contractants désignés:
**AT BE BG CH CY CZ DE DK EE ES FI FR GB GR HU IE IS IT LI LT LU MC NL PL PT RO SE SI SK TR**

(30) Priorité: **17.06.2004 FR 0406605**

(43) Date de publication de la demande:
**28.02.2007 Bulletin 2007/09**

(60) Demande divisionnaire:
**15186109.3**

(73) Titulaire: **Sensient Cosmetic Technologies
95130 Saint Ouen L'Aumone (FR)**

(72) Inventeurs:
- **SEU-SALERNO, Martine, c/o LCW F-95310 Saint-Ouen L'Aumone (FR)**
- **GRIZZO, Serge, c/o LCW F-95310 Saint-Ouen L'Aumone (FR)**

(74) Mandataire: **Colombet, Alain André et al
Cabinet Lavoix
2, Place d'Estienne d'Orves
75441 Paris Cedex 09 (FR)**

(56) Documents cités:

| | |
|---|---|
| **EP-A- 0 930 063** | **EP-A- 1 284 130** |
| **EP-A- 1 454 619** | **WO-A-03/013456** |
| **WO-A-03/013468** | **WO-A-03/020224** |
| **WO-A-03/075877** | **US-A- 6 136 299** |
| **US-B1- 6 555 095** | **US-B2- 6 491 901** |

- **LCW-Les colorants Wackerr-COVASORB-Technical Bulletin**
- **Solution in ethanol (10 mg/l) OMC70/BMDBM15/OS15 OMC20/BMDBM20/OS60**
- **CERTIFICAT D'ANALYSES/CERTIFICATE OF ANALYIS SENSIENT LOT: 26026 Référence: COVASORB Date de fabrication/Manufcaturing date: 03.03.2003**
- **Happi: Household & Personal Products Industry, 1 January 2001 (2001-01-01), & The Free Library: Household & Personal Products Industry, 1 January 2001 (2001-01-01), Retrieved from the Internet: URL:http://www.thefreelibrary.com/CRODA+CR EATES+LIQUID+LAUNDRY+DETERGENT.-a070 391703 & The Free Library: "Reader Service", Household & Personal products Industry, 1 February 2001 (2001-02-01), ISSN: 0090-8878 Retrieved from the Internet: URL:http://www.thefreelibrary.com/_/print/PrintArticle.aspx?id=71762716 & SOFW JOURNAL: "Dow Corning introduces Sensigel: The new perfume product category", SOFW JOURNAL, vol. 126, no. 9, 2000, page 92,**
- **THE FREE LIBRARY: HOUSEHOLD & PERSONAL PRODUCTS INDUSTRY, 1 February 2001 (2001-02-01), Retrieved from the Internet: URL: http://www.thefreelibrary.com/CONDEA+V ISTA+DEVELOPS+SPF+SUN+SPRAY.-a0717627 02**
- **Technical Data Sheet-"Rouge Covasorb W 3784 A" SENSIENT Cosmetic technologies**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **LEIBOWITZ M.R. ET AL: "ULTRAVIOLET ABSORBERS AS STABILIZERS IN THE COSMETIC INDUSTRY", JOURNAL OF THE SOCIETY OF COSMETIC CHEMISTS, 1 January 1963 (1963-01-01), pages 217-225, XP055148603,**

Remarques:

Le dossier contient des informations techniques présentées postérieurement au dépôt de la demande et ne figurant pas dans le présent fascicule.

## Description

La présente invention a trait à l'utilisation de compositions pour stabiliser la coloration de formulations, notamment de formulations cosmétiques.

En particulier dans le domaine de la cosmétique, de nombreuses formulations comprennent des colorants, naturels ou synthétiques, qui sont généralement destinés à en améliorer l'aspect et/ou à leur conférer une coloration caractéristique. Une coloration peut également apparaître en raison de la coloration intrinsèque de certains ingrédients, par exemple des parfums.

Dans les deux cas, la coloration de la formulation est due à la présence de composés absorbant la lumière de manière sélective.

Souvent, de telles formulations colorées sont conditionnées dans des emballages transparents à la lumière, par exemple en verre ou en matière plastique, de façon à laisser apparaître les produits. Ces emballages sont généralement également transparents aux rayons ultraviolets (UV).

Or étant des composés qui absorbent la lumière, les colorants sont sensibles à la lumière et plus particulièrement aux rayons ultraviolets particulièrement énergétiques. En effet, l'exposition à des rayons ultraviolets peut induire des réactions radicalaires modifiant la structure chimique des colorants et de ce fait leurs propriétés colorantes. On observe alors une modification de la couleur des formulations, sous forme de décoloration et/ou d'un virement de couleur.

Pour améliorer la stabilité des formulations colorées vis-à-vis des rayons ultraviolets, on peut utiliser des filtres ultraviolets, qui assurent une protection par absorption sélective du rayonnement UV.

A titre de filtres ultraviolets, on a utilisé notamment des composés de la famille des benzophénones.

Ces composés présentent toutefois certains inconvénients. D'une part, ils présentent une coloration jaune prononcée. Cette coloration propre s'ajoute à la coloration initiale, par exemple en transformant une coloration bleue initiale en coloration verte.

De plus, l'efficacité des benzophénones en matière de stabilisation de la coloration s'est révélée insuffisante. En outre, les molécules de type benzophénone sont en général mal adaptées à une utilisation dans des compositions cosmétiques aqueuses. En effet, les benzophénones sont généralement non hydrosolubles, et/ou présentent un caractère acide marqué, ce qui implique une étape supplémentaire d'ajustement du pH de la composition lorsqu'un tel composé est utilisé.

D'autres composés absorbant les rayons ultraviolets sont connus, tels que ceux mentionnés par exemple dans l'annexe VII de la Directive C.E.E. N° 76/168 (relative aux filtres ultraviolets acceptés en cosmétique) ou dans les Directives de la F.D.A. (Food and Drugs Administration américaine), à savoir dans le Federal Register, vol. 43, n° 166 (25 août 1978).

Ces composés sont toutefois destinés à protéger la peau et les cheveux contre les effets nocifs des rayons UV, et non pas les produits cosmétiques eux-mêmes ou leurs constituants, comme les colorants. A ce sujet, on pourra notamment se reporter à l'article "Filtres et écrans solaires" de M.C. Poelman dans «Actifs et additifs en cosmétologie », 2ème édition, 1999, Editions Tec & Doc, Paris.

En effet, des travaux conduits par les inventeurs ont permis d'établir que l'utilisation d'un seul filtre UV dans une formulation n'assure pas toujours une protection efficace de la coloration vis-à-vis des rayons ultraviolets.

Ainsi, la présence d'un filtre UV dans une composition cosmétique ne suffit pas à stabiliser la coloration vis-à-vis des rayons UV, alors qu'il permet de protéger la peau ou les cheveux lorsque la composition y est appliquée.

La présente invention a donc pour but l'utilisation d'une composition pour assurer la stabilisation effective et durable de la coloration contre les effets néfastes des rayonnements ultraviolets et ne présentant pas les inconvénients mentionnés ci-dessus.

Dans ce cadre, l'invention se fixe en particulier pour but de fournir des compositions stabilisantes compatibles avec une utilisation en cosmétique.

A cet effet, la présente invention propose l'utilisation d'une composition stabilisante consistant en :

- une association de:

  (A) 70% en masse d'un filtre ultraviolet de la famille des cinnamates (désigné ci-après par "composé A") ; et
  (B) 15% en masse d'un filtre ultraviolet de la famille des dibenzoylméthanes (désigné ci-après par "composé B") ; et
  (C) 15% en masse d'un filtre ultraviolet de la famille des salicylates (désigné ci-après par "composé C") ;

  et
- éventuellement un tensioactif ;

à titre d'additif stabilisateur de la coloration d'une formulation, ladite composition se présentant sous forme liquide, les

pourcentages en masse étant des pourcentages en masse par rapport à la masse totale des composés A, B et C.

Par "filtre ultraviolet" (ou "filtre UV"), on entend, au sens de la présente description, un composé chimique capable d'absorber des rayonnements électromagnétiques dans une gamme comprise dans le domaine des rayonnements ultraviolets, à savoir dans le domaine de longueurs d'ondes comprises entre 4 et 400 nm. L'association des composés A, B et C permet en général une absorption des rayonnements compris entre 200 et 400 mm.

Les filtres UV de la famille des cinnamates, des dibenzoylméthanes et des salicylates sont des composés bien connus qui sont décrits notamment dans la Directive C.E.E. N° 76/168 (filtres ultraviolets acceptés en cosmétique) ou dans les Directives de la F.D.A. (Food and Drugs Administration américaine), à savoir dans le Federal Register, vol. 43, n° 166 (25 août 1978).

En effet, les inventeurs ont constaté que, de façon surprenante l'association des composés A, B et C assure une stabilisation très efficace des colorants d'une formulation aux rayonnements ultraviolets.

Cette association est d'autant plus intéressante qu'elle est incolore ou très faiblement colorée.

Avantageusement, l'association des composés A, B et C est adaptée à une utilisation dans une formulation cosmétique, chacun des composés A, B et C étant un filtre UV autorisé dans le domaine cosmétique, notamment selon les législations européenne, américaines et japonaises.

Le composé A est un filtre ultraviolet de la famille des cinnamates répondant à la formule générale (I) suivante :

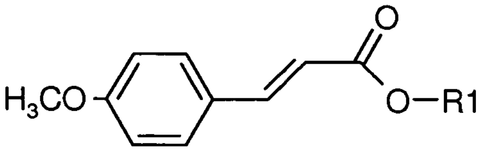

(I)

dans laquelle R1 représente un groupement alkyle, linéaire ou ramifié, comprenant 2 à 18 atomes de carbone, en particulier 3 à 16 atomes de carbone, notamment 2-éthyl-hexyle, $CH_2$-$CH(C_4H_9)(C_2H_5)$, ou isopentyle.

En général, dans la composition de l'invention, un unique ester de l'acide cinnamique est présent à titre de composé A. Toutefois, selon un mode de réalisation particulier, le composé A comprend un mélange d'au moins deux esters de l'acide cinnamique distincts.

De façon préférentielle, le composé A est choisi parmi :

- le 2-éthyl-hexyl-méthoxycinnamate ("méthoxy-cinnamate d'octyle" ou "octinoxate"), ;
- le glycéryl-éthyl-hexanoate-diméthoxy-cinnamate ("glycéryl octanate di-p-méthoxycinnamate") ;
- l'isopropyl-méthoxy-cinnamate;
- le diisopropyl-méthyl-cinnamate ;
- l'isopentyl-4-méthoxy-cinnamate ("isoamyle p-méthoxy-cinnamate")
- le diéthanolamine méthoxycinnamate ;

seuls ou en mélange, tel que par exemple le mélange d'isopropyl-méthoxy-cinnamate et de diisopropyl-cinnamate.

Avantageusement, le composé A est le 2-éthyl-hexyl-méthoxycinnamate.

Le composé B est un filtre UV de la famille des dibenzoylméthanes répondant à la formule générale (II) suivante :

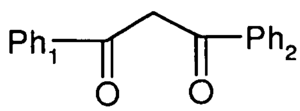

(II)

dans laquelle $Ph_1$ et $Ph_2$, identiques ou différents, représentent un groupe aryle mono- ou di-substitué (en particulier en position para ou ortho et para), par un groupe hydroxy, alkyle linéaire ou ramifié en $C_1$ à $C_{12}$ ou alkoxy en $C_1$ à $C_6$.

De préférence, le composé B répond à la formule (IIa) suivante :

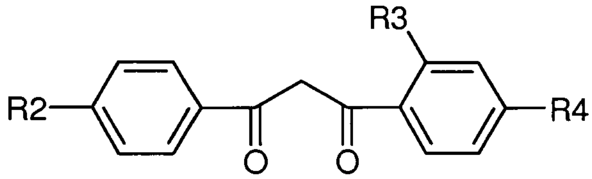

(IIa)

dans laquelle R2, R3 et R4, identiques ou différents, représentent un groupe alkyle en $C_1$ à $C_{12}$, de préférence en $C_2$ à $C_8$, et en particulier méthyle, éthyle, propyle ou butyle ou un groupe alkoxy, en particulier méthoxy ou éthoxy.

De façon particulièrement avantageuse, le composé B est le butyl-méthoxy-dibenzoylméthane (dit aussi "avobenzone").

En général, dans la composition de l'invention, un unique composé de formule (II) est présent à titre de composé B. Il est cependant envisageable que le composé B comprenne un mélange d'au moins deux composés de formule (II) distincts.

Enfin, le composé C est un filtre ultraviolet de la famille des salicylates répondant à la formule (III) suivante :

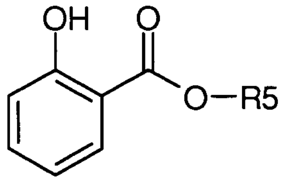

(III)

où R5 désigne un groupe alkyle linéaire ou ramifié en $C_2$ à $C_{12}$, de préférence de $C_3$ à $C_8$, un groupe arylalkyle en $C_7$ à $C_{10}$ éventuellement substitué par un groupe alkyle, une amine éventuellement substituée, un groupe hydroxy ou alkoxy en $C_1$ à $C_6$ éventuellement substitué.

En général, dans la composition de l'invention, un unique ester de formule (III) est présent à titre de composé C. Il est toutefois envisageable, que le composé C comprenne un mélange d'au moins deux esters de formule (III) distincts.

Le composé C est avantageusement choisi parmi :

- l'éthyl-hexyl-salicylate ("octylsalicylate" ou "octisalate") ;
- la triéthanolamine salicylate (TEA-salicylate) ;
- le dipropyléneglycol salicylate ;
- le salicylate d'isopropyl-4-benzyle ;

seuls ou en mélange.

Le composé C est avantageusement l'éthyl-hexyl-salicylate.

Selon l'invention, la composition stabilisante se présente sous forme liquide, ce qui facilite le dosage et permet une incorporation efficace et homogène dans la formulation.

Il est décrit que le composé A peut être l'hexyl-méthoxy-cinnamate, le composé B peut être le butyl-méthoxy-dibenzoylméthane et le composé C peut être l'éthyl-hexyl-salicylate.

Dans la composition, le composé A est de préférence l'éthyl-hexyl-méthoxy-cinnamate, le composé B est le butyl-méthoxy-benzoyl-méthane, et le composé C l'éthyl-hexyl-salicylate

Il est décrit que quelle que soit la nature exacte des constituants A, B et C, la composition stabilisante peut se présenter sous différentes formes en fonction de la destination envisagée.

Les composés A, B et C sont solubles dans la plupart des solvants organiques, notamment dans les alcools et dans les phases lipidiques. De ce fait, la composition stabilisante sous cette forme simple aisément incorporée dans les compositions cosmétiques comportant des solvants organiques ou des tensioactifs telles que les shampooings, les bains moussants, les eaux de toilette, eaux de parfums et huiles.

La composition stabilisante peut toutefois aussi être solubilisée dans des formulations à base de solvants hydrophiles, et notamment dans des formulations aqueuses en présence d'un tensioactif adapté.

Selon une variante intéressante, la composition stabilisante comporte donc outre les composés A, B et C un tensioactif.

Des tensioactifs particulièrement adaptés sont notamment les tensioactifs non ioniques et en particulier le mélange de tensioactifs non ioniques commercialisé par la société LCW sous le nom de solubilisant LRI.

Le tensioactif est mis en oeuvre dans une quantité suffisante pour assurer la solubilisation de la composition

stabilisante dans la formulation. Généralement, une quantité de 20 à 40%, et de préférence de l'ordre de 30% en masse par rapport au total des composés A + B + C est appropriée.

Selon cette variante, la composition stabilisante ajoutée à la formulation est solubilisée en formant une émulsion ou micro-émulsion, dans laquelle la phase huileuse comprend les composés A, B et C.

Les compositions stabilisantes décrites permettent de stabiliser la coloration d'une formulation comportant ou non une phase organique. Ainsi, la formulation stabilisée peut être de type solution, émulsion, ou gel et comportant des colorants naturels ou artificiels, hydrosolubles ou liposolubles.

Dans ce cadre, l'invention a en particulier pour objet l'utilisation des compositions stabilisantes précitées pour stabiliser les colorants dans des formulations cosmétiques, telles que des parfums, des eaux de toilette, ou encore des produits de soin ou d'hygiène corporelle tels que des shampooings ou des gels douches, par exemple.

Les utilisations précitées concernent en particulier la stabilisation des colorants sensibles au rayonnement ultraviolet, notamment les colorants organiques tels que le F&DC Red 40, les colorants naturels, comme la garance, le carmin soluble, le caramel, la chlorophylle ou le curcumin.

Afin de préserver la coloration des effets des rayons UV, on ajoute la composition stabilisante à la formulation en une quantité appropriée. Cette quantité dépendra de la nature des colorants, mais aussi du degré d'exposition de la formulation aux rayons UV. Elle sera donc notamment fonction de la transparence du récipient aux rayons UV et de la durée de vie de la formulation.

En principe, une teneur de 0,1 à 3%, de préférence de 0,2% à 2%, et tout particulièrement de 0,5% à 1,5% en masse par rapport à la masse totale de la formulation est suffisante.

Dans le cas particulier de composition aqueuse, il est en général préférable que le mélange des composés A, B et C soit mis en oeuvre à raison de 1,5 à 4% en masse par rapport à la masse totale de la formulation stabilisée.

Il est décrit des formulations, notamment cosmétiques, comprenant la composition stabilisante telle que définie précédemment.

Il est décrit que la formulation peut comprendre une phase huileuse telle que, par exemple, une huile polaire, une huile apolaire ou un parfum, ou bien encore une phase hydro-alcoolique, comprenant au moins 10%, voire au moins 30% en masse d'un alcool tel que l'éthanol.

Il est décrit que si la formulation comprend une phase aqueuse ou hydro-alcoolique, la composition stabilisante peut être solubilisée grâce à un tensioactif. Le tensioactif peut être déjà présent dans la formulation, ou dans la composition stabilisante ajoutée.

**Mesures colorimétriques**

L'effet stabilisant de la composition est mis en évidence par les mesures colorimétriques suivantes.

On a préparé des solutions des colorants FD&C Blue 1 et FD&C Red 40 dans de l'eau distillée, lesquelles ont été diluées afin d'obtenir une absorbance comprise entre 0,3 et 1. On divise la solution obtenue en deux parties égales, dont une sert de témoins alors que l'autre est exposée à la lumière solaire dans un appareil SUN-TEST de chez Heraeus pendant 72 heures.

Ensuite, on enregistre le spectre d'absorbance de l'échantillon et du témoin de chaque colorant au moyen d'un spectrophotomètre UV-Visible (Varian, Modèle CARY 1) entre 200 et 720 nm. A partir de ces spectres, on détermine les valeurs respectives d'absorbance $A_{max}$ aux maxima d'absorbance pour le témoin et l'échantillon respectivement.

La force colorante de l'échantillon X est alors donnée par la relation suivante :

$$X = \frac{A_{\max(témoin)}}{A_{\max(échantillon)}} x100$$

Un des paramètres permettant de caractériser la coloration d'une formulation est son absorption à une longueur d'onde caractéristique. De préférence, l'absorbance caractéristique dans les formulations comprenant la composition stabilisante après exposition à la lumière pour une durée d'un mois chute de 15% au plus, encore préférée de 10% au plus et tout particulièrement de 8% au plus.

D'autres avantages et caractéristiques de la présente invention apparaîtront de façon plus claire au vu des exemples illustratifs exposés ci-après.

**<u>EXEMPLE 1 comparatif</u> : Préparation d'une composition stabilisante**

On a réalisé une composition stabilisante comprenant 80% en masse d'éthyl-hexyl-méthoxy-cinnamate, 10% en masse de butyl-méthoxy-benzoyl-méthane, et 10% en masse d'éthyl-hexyl-salicylate selon le protocole ci-après.

Dans un mélangeur, on a introduit 80g d'éthyl-hexyl-méthoxy-cinnamate10 g de butyl-méthoxy-dibenzoyl-méthane, et10 g d'éthyl-hexyl-salicylate.
Le milieu ainsi obtenu a été chauffé à 60° C sous agitation jusqu'à obtention d'un liquide clair.

**EXEMPLE 2 : Préparation d'une composition stabilisante**

On a réalisé une composition stabilisante selon le protocole ci-après.
Dans un mélangeur, on a introduit 70g d'éthyl-hexyl-méthoxy-cinnamate, 15g de butyl-méthoxy-dibenzoyl-méthane, et 15g d'éthyl-hexyl-salicylate.

**EXEMPLE 3 comparatif : Préparation d'une composition stabilisante**

On a réalisé une composition stabilisante selon le protocole ci-après.
Dans un mélangeur, on a introduit 60g d'éthyl-hexyl-méthoxy-cinnamate, 20g de butyl-méthoxy-dibenzoyl-méthane, et 20g d'éthyl-hexyl-salicylate.
Le milieu ainsi obtenu a été chauffé à 60° C sous agitation jusqu'à obtention d'un liquide clair.

**EXEMPLE comparatif: Préparation d'une composition stabilisante**

On a réalisé une composition stabilisante selon le protocole suivant.
Dans un mélangeur on a introduit 90g de butyl-méthoxy-benzoyl-méthane, puis on a introduit 5g d'éthyl-méthoxy-cinnamate et 5g d'éthyl-hexyl-salicylate. On a obtenu, après malaxage d'une durée de 2 minutes un mélange des trois composés se présentant sous la forme d'une poudre.
Les compositions des exemples 1 à 4 sont reportées dans le tableau 1 ci-après, où les pourcentages indiqués sont exprimés par rapport à la masse totale des composés A (éthyl-hexyl-méthoxy-cinnamate), B (butyl-méthoxy-benzoyl-méthane), C (éthyl-hexyl-salicylate).

Tableau 1 : Composition stabilisante des exemples 1 à 4

| | Exemple 1 | Exemple 2 | Exemple 3 | Exemple 4 |
|---|---|---|---|---|
| Composé A Ethyl-hexyl-méthoxycinnamate | 80% | 70% | 60% | 5% |
| Composé B Butyl-méthoxy-benzoyl-méthane | 10% | 15% | 20% | 90% |
| Composé C Ethyl-hexyl-salicylate | 10% | 15% | 20% | 5% |

**EXEMPLE 5 : Formulations comprenant la composition stabilisante**

Les compositions stabilisantes des exemples 1 à 4 précédents ont été ajoutées à différentes formulations cosmétiques.

**a- Shampooings.**

On a réalisé des formulations de shampooings a1 à a5 selon les indications du tableau 2 ci-dessous, en ajoutant à la fin 1 % en masse par rapport à la formulation finie de composition stabilisante préparée dans les exemples précédents.

Tableau 2 : Formulations de shampooings

| | a1 | a2 | a3 | a4 | a5 (témoin) |
|---|---|---|---|---|---|
| Texapon NSO [sodium lauryl éther sulfate] | 30% | 30% | 30% | 30% | 30% |
| Cocoamidopropyl-bétaïne | 10% | 10% | 10% | 10% | 10% |
| Colorant rouge FD&C Red 40 | 0,001% | 0,001% | 0,001% | 0,001% | 0,001% |
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |

(suite)

| | a1 | a2 | a3 | a4 | a5 (témoin) |
|---|---|---|---|---|---|
| Eau | q.s. 100% | q.s. 100% | q.s. 100% | q.s. 100% | q.s. 100% |
| pH | 4,7 | 5,2 | 4,6 | 4,7 | 5,7 |

**b - Compositions huileuses**

On a réalisé des compositions huileuses b1 à b5 selon les indications du tableau 3 ci-dessous, en ajoutant à la fin 1 % en masse par rapport à la formulation finie de composition stabilisante préparée dans les exemples précédents.

Tableau 3 : Compositions huileuses

| | b1 | b2 | b3 | b4 | b5 (témoin) |
|---|---|---|---|---|---|
| Colorant rouge D&C Red 17 | 0,001% | 0,001% | 0,001% | 0,001% | 0,001% |
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |
| Huile de Ricin | q.s. 100% | q.s. 100% | q.s. 100% | q.s. 100% | q.s. 100% |

**c - Eaux de toilette**

On a réalisé des compositions eaux de toilette c1 à c5 selon les indications du tableau 4 ci-dessous, en ajoutant au parfum 1% en masse par rapport à la formulation finie de composition stabilisante préparée dans les exemples précédents puis le colorant et l'éthanol.

Tableau 4 : Eaux de toilette

| | c1 | c2 | c3 | c4 | c5 (témoin) |
|---|---|---|---|---|---|
| Parfum | 5% | 5% | 5% | 5% | 5% |
| Colorant rouge FD&C Red 40 | 0,001% | 0,001% | 0,001% | 0,001% | 0,001% |
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |
| Ethanol 96° | q.s. 100% | q.s. 100% | q.s. 100% | q.s. 100% | q.s. 100% |

**d - Compositions aqueuses**

On a réalisé différentes compositions aqueuses d1 à d4 dont les compositions sont reportées dans le tableau 5 ci-après. La composition stabilisante préparée selon les exemples 1 à 4 est mélangée au préalable avec un mélange de tensioactifs non ioniques (solubilisant LRI commercialisé par la société LCW) dans un ratio pondéral de 1:2. Le mélange est ensuite chauffé à 70° C sous agitation jusqu'à obtention d'une solution limpide.

Dans le cas de la composition stabilisante selon l'exemple 4, la solution devient translucide et granuleuse lorsqu'elle est refroidie sous agitation.

Tableau 5 : Compositions aqueuses

| | d1 | d2 | d3 | d4 | d5 (témoin) |
|---|---|---|---|---|---|
| Colorant bleu FD&C Blue 1 | 0,001% | 0,001% | 0,001% | 0,001% | 0,001% |

(suite)

| | d1 | d2 | d3 | d4 | d5 (témoin) |
|---|---|---|---|---|---|
| Composition de l'exemple 1 | 1% | - | - | - | - |
| Composition de l'exemple 2 | - | 1% | - | - | - |
| Composition de l'exemple 3 | - | - | 1% | - | - |
| Composition de l'exemple 4 | - | - | - | 1% | - |
| Solubilisant LRI | 2% | 2% | 2% | 2% | - |
| Eau | q.s. 100% | q.s. 100% | q.s. 100% | q.s. 100% | q.s. 100% |
| pH | 4,7 | 5,2 | 4,6 | 4,7 | 5,7 |

Les différentes compositions réalisées ont été exposées à des rayonnements ultraviolets pendant 72 heures dans un appareil SUN TEST. A l'issue de ce traitement, on a mesuré la force colorante comme décrit ci-dessus.

Les résultats sont exposés dans les tableaux 6 à 9 ci-après. Ils ont été évalués comme suit :

| | |
|---|---|
| chute de 0%-20% de la force de coloration | ++++ |
| chute de 21-40% de la force de coloration | +++ |
| chute de 41-60% de la force de coloration | ++ |
| chute de 61-80% de la force de coloration | + |
| chute de 81-100% de la force de coloration | 0 |

Tableau 6 : Shampooings

| Compositions | a1 | a2 | a3 | a4 | a5 (témoin) |
|---|---|---|---|---|---|
| Stabilisation observée | ++++ | ++ | + | ++ | 0 |

Tableau 7 : Compositions huileuses

| Compositions | b1 | b2 | b3 | b4 | b5 (témoin) |
|---|---|---|---|---|---|
| Stabilisation observée | +++ | +++ | +++ | ++ | 0 |

Tableau 8 :Eaux de toilette

| Compositions | c1 | c2 | c3 | c4 | c5 (témoin) |
|---|---|---|---|---|---|
| Stabilisation observée | ++++ | ++++ | +++ | ++++ | 0 |

Tableau 9 : Compositions aqueuses

| Compositions | d1 | d2 | d3 | d4 | d5 (témoin) |
|---|---|---|---|---|---|
| Stabilisation observée | +++ | ++++ | +++ | ++++ | 0 |

Ces résultats montrent clairement un très net effet de stabilisation obtenu lorsque l'on introduit la composition stabilisante de l'exemple 2 dans les différentes formulations.

**EXEMPLE COMPARATIF**

A titre de comparaison, on a réalisé des compositions similaires à celles de l'exemple 5, dans lesquelles on a remplacé les compositions stabilisantes par des composés A, B et C pris isolément. Les compositions ont été testées

dans les mêmes conditions que dans l'exemple 5 (exposition aux rayonnements ultraviolets pendant 72 heures).

Les résultats observés sont exposés dans les tableaux 10 à 13 ci-après, d'où il ressort que, dans toutes les compositions testées, la présence du composé A, B ou C seule ne permet pas de stabiliser les colorants. Au contraire, on observe même une dégradation du comportement de la composition sous le rayonnement ultraviolet.

**Tableau 10 : Compositions de shampooings**

| | a1' | a2' | A3' | a5' |
|---|---|---|---|---|
| Texapon NSO (sodium lauryl éther sulphate) | 30% | 30% | 30% | 30% |
| Cocoamidopropyl-bétaine | 10% | 10% | 10% | 10% |
| Colorant rouge : FD&C Red 40 | 0,001% | 0,001% | 0,001% | 0,001% |
| Composé A Ethyl-hexyl-méthoxycinnamate | 1% | | | |
| Composé B Butyl-méthoxy-benzoyl-méthane | | 1% | | |
| Composé C Ethyl-hexyl-salicylate | | | 1% | |
| Eau | q.s. 100% | q.s. 100% | q.s. 100% | q.s. 100% |
| Stabilité vis-à-vis des UV | 0 | 0 | 0 | 0 |

**Tableau 11 : Compositions huileuses**

| | b1' | b2' | b3' | b5' |
|---|---|---|---|---|
| Colorant rouge : D&C Red 17 | 0,001% | 0,001% | 0,001% | 0,001% |
| Composé A Ethyl-hexyl-méthoxycin namate | 1% | | | |
| Composé B Butyl-méthoxy-benzoyl-méthane | | 1% | | |
| Composé C Ethyl-hexyl-salicylate | | | 1% | |
| Huile de ricin | q.s. 100% | q.s. 100% | q.s. 100% | q.s. 100% |
| Stabilité vis-à-vis des UV | 0 | 0 | 0 | 0 |

**Tableau 12 : Eaux de toilette**

| | c1' | c2' | c3' | c5' |
|---|---|---|---|---|
| Parfum | 5% | 5% | 5% | 5% |
| Colorant rouge : FD&C Red 40 | 0,001% | 0,001% | 0,001% | 0,001% |
| Composé A Ethyl-hexyl-méthoxycin namate | 1% | | | |
| Composé B Butyl-méthoxy-benzoyl-méthane | | 1% | | |
| Composé C Ethyl-hexyl-salicylate | | | 1% | |
| Ethanol 96° | q.s. 100% | q.s. 100% | q.s. 100% | q.s. 100% |
| Stabilité vis-à-vis des UV | 0 | 0 | 0 | 0 |

**Tableau 13 : Compositions aqueuses**

| | d1' | d2' | d3' | d5' |
|---|---|---|---|---|
| Colorant bleu : FD&C Blue 1 | 0,001% | 0,001% | 0,001% | 0,001% |
| Composé A Ethyl-hexyl-méthoxycinnamate + solubilisant LRI | 3% | | | |
| Composé B Butyl-méthoxy-benzoyl-méthane + solubilisant LRI | | 3% | | |

(suite)

| | d1’ | d2’ | d3’ | d5’ |
|---|---|---|---|---|
| Composé C Ethyl-hexyl-salicylate + solubilisant LRI | | | 3% | |
| Eau distillée | q.s. 100% | q.s. 100% | q.s. 100% | q.s. 100% |
| Stabilité vis-à-vis des UV | ++ | + | ++ | ++ |

**Revendications**

1. Utilisation d’une composition consistant en :

   - une association de :

     (A) 70% en masse d’un filtre ultraviolet de la famille des cinnamates, désigné ci-après par composé A ; et
     (B) 15% en masse d’un filtre ultraviolet de la famille des dibenzoylméthanes, désigné ci-après par composé B ; et
     (C) 15% en masse d’un filtre ultraviolet de la famille des salicylates, désigné ci-après par composé C,

   et
   - éventuellement un tensioactif ;

   à titre d’additif stabilisateur de la coloration d’une formulation, ladite composition se présentant sous forme liquide, les pourcentages en masse étant des pourcentages en masse par rapport à la masse totale des composés A, B et C.

2. Utilisation selon la revendication 1, **caractérisée en ce que** le composé A est un composé répondant à la formule générale (I) suivante :

   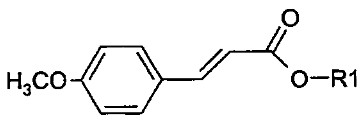

   **(I)**

   dans laquelle R1 représente un groupe alkyle linéaire ou ramifié comportant 2 à 18, de préférence 3 à 16 atomes de carbone.

3. Utilisation selon la revendication 2, **caractérisée en ce que** le composé A est le 2-éthyl-hexyl-méthoxycinnamate.

4. Utilisation selon l’une des revendications 1 à 3, **caractérisée en ce que** le composé B est un composé répondant à la formule générale (II) suivante :

   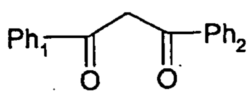

   **(II)**

   (H) dans laquelle $Ph_1$ et $Ph_2$, identiques ou différents, représentent un groupe aryle mono- ou di-substitué par un groupe hydroxy, alkyle linéaire ou ramifié en $C_1$ à $C_{12}$ ou alkoxy en $C_1$ à $C_6$.

5. Utilisation selon la revendication 4, **caractérisée en ce que** le composé B est un composé répondant à la formule (IIa) suivante :

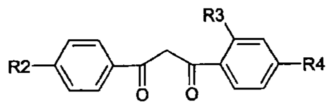

(IIa)

(IIa) dans laquelle R2, R3 et R4, identiques ou différents, représentent chacun, indépendamment, un groupement alkyle en $C_1$ à $C_{12}$.

6. Utilisation selon la revendication 5, **caractérisée en ce que** le composé B est le butyl-méthoxy-dibenzoylméthane.

7. Utilisation selon l'une des revendications 1 à 6, **caractérisée en ce que** le composé C est un composé répondant à la formule (III) suivante :

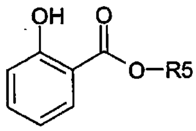

(III)

où R5 désigne un groupe alkyle en $C_2$ à $C_{12}$.

8. Utilisation selon la revendication 7, **caractérisée en ce que** le composé (C) est l'éthyl-hexyl-salicylate.

9. Utilisation selon l'une quelconque des revendications précédentes, **caractérisée en ce que** la composition comprend un tensioactif.

10. Utilisation selon l'une quelconque des revendications précédentes, dans laquelle la formulation est une formulation cosmétique.

**Patentansprüche**

1. Verwendung einer Zusammensetzung, bestehend aus:

   - einer Verbindung von:

     (A) 70 Massen-% eines ultravioletten Filters der Familie der Cinnamate, im Folgenden als Verbindung A bezeichnet; und
     (B) 15 Massen-% eines ultravioletten Filters der Familie der Dibenzoylmethane, im Folgenden als Verbindung B bezeichnet; und
     (C) 15 Massen-% eines ultravioletten Filters der Familie der Salicylate, im Folgenden als Verbindung C bezeichnet,

   und
   - gegebenenfalls ein Tensid;

   als Stabilisatoradditiv der Färbung einer Formulierung, wobei die Zusammensetzung eine flüssige Form aufweist und die Massenprozente in Bezug auf die Gesamtmasse der Verbindungen A, B und C sind.

2. Verwendung nach Anspruch 1, **dadurch gekennzeichnet, dass** die Verbindung A eine Verbindung ist, die der folgenden allgemeinen Formel genügt:

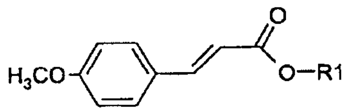

(I)

bei der R1 eine lineare oder verzweigte Alkylgruppe darstellt, die 2 bis 18, vorzugsweise 3 bis 16 Kohlenstoffatome aufweist.

3. Verwendung nach Anspruch 2, **dadurch gekennzeichnet, dass** die Verbindung A das 2-Ethylhexylmethoxycinnamat ist.

4. Verwendung nach einem der Ansprüche 1 bis 3, **dadurch gekennzeichnet, dass** die Verbindung B eine Verbindung ist, die der folgenden allgemeinen Formel (II) genügt:

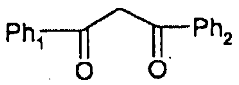

(II)

(H) bei der $Ph_1$ und $Ph_2$, identisch oder unterschiedlich, eine Arylgruppe, mono- oder disubstituiert durch eine Hydroxygruppe, eine lineare oder verzweigte $C_1$ bis $C_{12}$ Alkylgruppe oder eine $C_1$ bis $C_6$Alkoxygruppe darstellen.

5. Verwendung nach Anspruch 4, **dadurch gekennzeichnet, dass** die Verbindung B eine Verbindung ist, die der folgenden Formel (IIa) genügt:

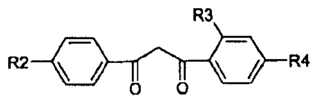

(IIa)

(IIa) bei der R2, R3 und R4, identisch oder unterschiedlich, jeweils unabhängig eine $C_1$ bis $C_{12}$ Alkylgruppe darstellen.

6. Verwendung nach Anspruch 5, **dadurch gekennzeichnet, dass** die Verbindung B das Butylmethoxydibenzoylmethan ist.

7. Verwendung nach einem der Ansprüche 1 bis 6, **dadurch gekennzeichnet, dass** die Verbindung C eine Verbindung ist, die der Formel (III) genügt:

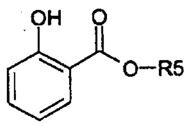

(III)

in der R5 eine $C_2$ bis $C_{12}$ Alkylgruppe bezeichnet.

8. Verwendung nach Anspruch 7, **dadurch gekennzeichnet, dass** die Verbindung (C) das Ethylhexylsalicylat ist.

9. Verwendung nach einem beliebigen der vorhergehenden Ansprüche, **dadurch gekennzeichnet, dass** die Zusammensetzung ein Tensid umfasst.

10. Verwendung nach einem beliebigen der vorhergehenden Ansprüche, bei der die Formulierung eine kosmetische Formulierung ist.

## Claims

1. Use of a composition consisting of:

    • an association of:

        A) 70% mass of an ultraviolet filter from the cinnamate family, referred to hereafter as compound A; and
        B) 15% mass of an ultraviolet filter from the dibenzoylmethane family, referred to hereafter as compound B; and
        C) 15% mass of an ultraviolet filter from the silicylate family, referred to hereafter as compound C,

    and
    • if necessary, a surfactant;

    as additive for stabilising the colour of a formulation, wherein said composition is present in liquid form and the mass percents are mass percents in relation to the total mass of compounds A, B and C.

2. Use according to claim 1, **characterised in that** compound A is a compound having the following general formula (I):

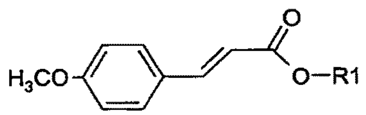

(I)

in which R1 represents a linear or branched alkyl group comprising 2 to 18, preferably 3 to 16 carbon atoms.

3. Use according to claim 2, **characterised in that** compound A is 2-ethylhexyl methoxycinnamate.

4. Use according to one of claims 1 to 3, **characterised in that** compound B is a compound having the following general formula (II):

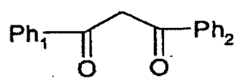

(II)

in which $Ph_1$ and $Ph_2$, identical or different, represent an aryl group mono- or disubstituted by a hydroxy group, linear or branched $C_1$-$C_{12}$ alkyl group or $C_1$-$C_6$ alkoxy group.

5. Use according to claim 4, **characterised in that** compound B is a compound having the following formula (IIa):

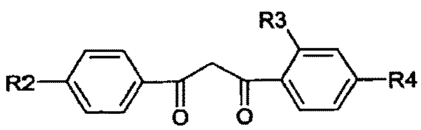

(IIa)

in which R2, R3 and R4, identical or different, each independently represent a $C_1$-$C_{12}$ alkyl group.

6. Use according to claim 5, **characterised in that** compound B is butyl methoxydibenzoylmethane.

7. Use according to one of claims 1 to 6, **characterised in that** compound C is a compound having the following formula (III):

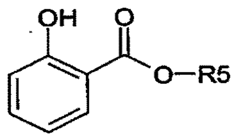

(III)

where R5 denotes a $C_2$-$C_{12}$ alkyl group.

**8.** Use according to claim 7, **characterised in that** compound (C) is ethylhexyl salicylate.

**9.** Use according to any one of the preceding claims, **characterised in that** the composition comprises a surfactant.

**10.** Use according to any one of the preceding claims, **characterised in that** the formulation is a cosmetic formulation.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- Food and Drugs Administration américaine. *Federal Register,* 25 Août 1978, vol. 43 (166
- Filtres et écrans solaires. **M.C. POELMAN.** Actifs et additifs en cosmétologie. Editions Tec & Doc, 1999